Europäisches Patentamt

(19) European Patent Office — (11) Publication number: **0 010 876**
B1

Office européen des brevets

(12) **EUROPEAN PATENT SPECIFICATION**

(45) Date of publication of patent specification: **08.12.82**

(21) Application number: **79302103.1**

(22) Date of filing: **04.10.79**

(51) Int. Cl.³: **A 61 M 31/00,**
**A 61 K 9/24, A 61 K 9/52,**
**A 61 J 3/06**

(54) Osmotically driven active agent dispenser.

(30) Priority: **23.10.78 US 953906**

(43) Date of publication of application:
**14.05.80 Bulletin 80/10**

(45) Publication of the grant of the patent:
**08.12.82 Bulletin 82/49**

(84) Designated Contracting States:
**CH DE FR GB IT**

(56) References cited:
**DE - A - 2 201 533**
**DE - A - 2 814 709**
**US - A - 3 995 631**
**US - A - 4 058 122**
**US - A - 4 111 202**
**US - A - 4 111 203**
**US - A - 4 116 241**

(73) Proprietor: **ALZA CORPORATION**
**950 Page Mill Road**
**Palo Alto, California 94304 (US)**

(72) Inventor: **Ayer, Atul D.**
**240 Monroe Drive**
**Mountain View, California 94040 (US)**
Inventor: **Theeuwes, Felix**
**1634 Fallen Leaf Lane**
**Los Altos, California 94022 (US)**

(74) Representative: **Evans, David Charles et al,**
**F.J. CLEVELAND & COMPANY 40-43, Chancery**
**Lane**
**London, WC2A 1JQ (GB)**

Courier Press, Leamington Spa, England.

Osmotically driven active agent dispenser

Technical field

This invention relates to an osmotically driven dispenser for dispensing osmotically effective active agent compositions, such as drug compositions, to water-containing environments, such as the human body.

Background art

Osmotically driven dispensers for dispensing an active agent to a water-containing environment of use are known. US—A—3,845,770 and 3,916,899 disclose dispensers that comprise a wall formed of a material permeable to water and impermeable to agent; a compartment defined by the wall that contains the agent; and an outlet passageway through the wall for dispensing the agent to the environment. These dispensers are remarkably effective for dispensing an agent that in solution exhibits an osmotic pressure greater than the osmotic pressure of the water in the environment or for delivering an agent that does not exhibit such osmotic pressure but which is admixed with a compound that does. An agent or compound which exhibits such a pressure is called an "osmotically effective" composition. The dispensers release agent by water being imbibed continuously through the wall into the compartment at a rate determined by the permeability of the wall to water and the osmotic pressure difference between the solution of osmotically effective composition and the water in the environment to generate hydrostatic pressure within the compartment and continuously produce a solution of the osmotically effective composition which is dispensed through the passageway to the environment.

According to US—A—3,845,770 and 3,916,899 the wall of such dispensers is made of a single layer of a homogeneous material. That material may either be substantially pure polymer or, as disclosed in US—A—4,111,202, a mixture of polymers and additives such as stabilizers. Further, US—A—4,160,452 discloses that the wall may be a laminate consisting of an inner semipermeable lamina and an outer microporous lamina.

Disclosure of invention

The present invention is an improvement of the osmotically driven dispensers disclosed in US—A—4,160,452 (which is equivalent to prior published DE—A1—2,814,709 in which (1) the wall of the dispenser includes a third layer that is between the inner semipermeable lamina and outer lamina, which may be microporous or hydrogel, and is composed of a water soluble composition, and (2) the passageway extends through the inner lamina from the active agent composition compartment to the third layer.

This improved dispenser operates as follows when placed in a water-containing environment. Water from the environment diffuses through the outer lamina and dissolves the water soluble composition that makes up the third layer, thus forming a liquid zone or layer between the inner lamina and outer lamina. The water soluble composition, in solution, diffuses outwardly from the liquid zone through the outer lamina while water from the zone diffuses inwardly through the inner semipermeable lamina into the active agent compartment of the dispenser. That water dissolves the active agent composition, thereby establishing an osmotic pressure imbalance between the liquid in said zone and the solution of active agent composition within the compartment. This imbalance causes water to be imbibed continuously through the semipermeable lamina into the compartment, which, in turn, establishes hydrostatic pressure within the compartment. That pressure continuously forces solution of active agent composition from the compartment into said liquid zone via the passageway through the inner lamina. Once in the liquid zone, the solution is diluted by the liquid in the zone. From said zone the active agent, in dilute solution, diffuses outwardly through the outer microporous or hydrogel lamina. In this manner, agent is released from the dispenser in a relatively dilute solution over the relatively large surface area defined by the exterior of the outer lamina.

Brief description of the drawings

In the drawings:

Figure 1 is an elevational side view of an embodiment of the invention for administering drug orally;

Figure 2 is an enlarged partly sectional embodiment of Figure 1 taken along line 2—2 in Figure 1;

Figure 3 is an enlarged, partly sectional view of the embodiment of Figure 1 showing schematically the operation of the embodiment once it is swallowed;

Figures 4 and 6 are graphs of the cumulative amounts of potassium chloride delivered by the dispensers of Examples 1 and 2, respectively; and

Figures 5 and 7 are graphs showing the average release rates of potassium chloride from the dispensers of Examples 1 and 2, respectively.

Detailed description of embodiments of invention

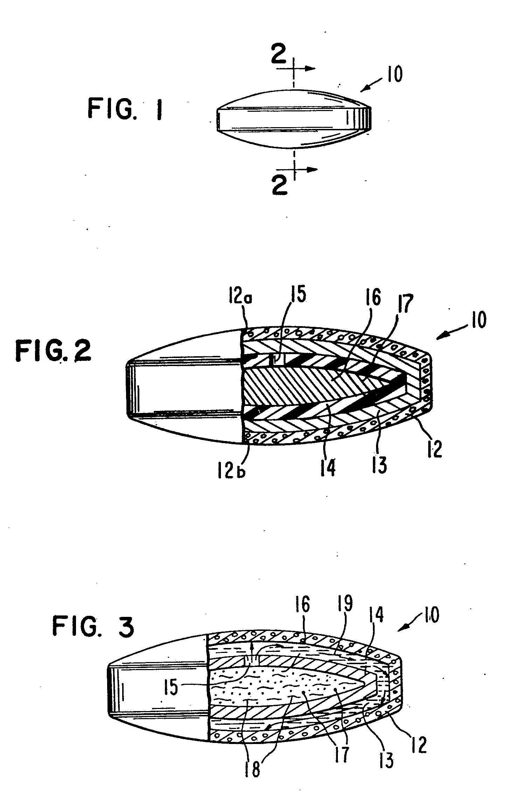

The dispenser of Figures 1—3 is generally designated 10. It is in the shape of a tablet and is intended to be administered orally. Refer to Figures 2 and 3, dispenser 10 includes: an outer microporous or hydrogel lamina 12, an intermediate water soluble compound lamina 13, and an inner semipermeable lamina 14 having a passageway 15 extending through it. Outer lamina 12 is removed from 12a to 12b to depict the inner structure of dispenser 10. Inner lamina 14 defines a central compartment 16 that is filled with a solid or semisolid osmotically effective drug formulation 17. Once dispenser 10 is swallowed, it becomes activated and commences dispensing drug formulation 17 to the gastrointestinal tract in the following manner. First, water from the tract diffuses through outer lamina 12 and dissolves intermediate water soluble layer 13. Lamina 13 is thus transformed from solid form (shown in Figure 2) to liquid form (shown in Figure 3). Then, water from lamina 13 diffuses through inner semipermeable lamina 14 and dissolves drug formulation 17 to form a solution 18 (represented by wavy lines in Figure 3) thereof. An osmotic pressure imbalance between solution 18 and liquid lamina 13 causes water to be imbibed through lamina 14 continuously. The imbibed water creates hydrostatic pressure within compartment 16 thereby causing solution 18 to be forced or pumped from compartment 16 via passageway 15. The solution 18 emerging from passageway 15 mixes with liquid lamina 13 and is thereby diluted and dispersed spatially within lamina 13 (indicated by flow lines 19 in Figure 3). From lamina 13 drug formulation 17 passes by diffusion through outer lamina 12 and into the gastrointestinal tract. The release of drug from dispenser 10 occurs at more diluted concentrations and over a greater surface area than in the prior art dispensers. Also, as discussed below, since lamina 13 becomes liquid *in vivo*, dispenser 10 may become softer and more compressible. This may make it more comfortable than the prior rigid dispensers for placement in body cavities such as the vagina and anus.

Embodiments of the invention dispenser that are intended for parenteral administration may be sized and shaped differently than dispenser 10, with the exact shape and size depending on the specific administration site. Also, embodiments that are intended to administer active agents other than drugs to environments other than animal bodies will be sized and shaped to accommodate the particular environment of use.

The release rate of drug 17 from dispenser 10 is given by the equation:

$$\frac{dm}{dt_t} = \frac{\pi S}{R_t} \qquad (1)$$

where $\pi$ is the osmotic pressure of saturated drug solution 18 in compartment 16, S is the solubility of the drug formulation 17 in water and $R_t$ is the combined water transport resistance of laminas 12, 13 and 14 and is given by the equation

$$R_t = R_1 + R_2 + R_3 \qquad (2)$$

where $R_1$ is the water transport resistance of lamina 14, $R_2$ is the water transport resistance of lamina 13 and $R_3$ is the water transport resistance of lamina 12. The water transport resistance, R, of a lamina is given by the equation

$$R = \frac{h}{KA} \qquad (3)$$

where h is the thickness of the lamina, K is its permeability to water and A is its area.

The hardness or softness (rigidity) of dispenser 10 depends on the hydrostatic pressure in liquid lamina 13. When that pressure is low, (close to atmospheric pressure) dispenser 10 feels soft and may be compressed easily. Vice versa, when such pressure is high (relative to atmospheric pressure) dispenser 10 feels hard and is not compressed easily. The rigidity of dispenser 10 may be predetermined and controlled as follows. The hydrostatic pressure, $\Delta P$, across outer lamina 12 is given by:

$$\Delta P = \pi(\pi_2 - \pi_e) \qquad (4)$$

where $\pi$ is the reflection coefficient of lamina 12, $\pi_2$ is the osmotic pressure of the liquid composing lamina 13 and $\pi_e$ is the osmotic pressure of the gastrointestinal fluid. $\pi_2$ may be calculated from Van T'Hoff's Law using the average concentration of drug 17 in liquid lamina 13. Thus, rigidity may be predetermined by selecting a material having a given reflection coefficient to form lamina 12. The reflection coefficient, which may vary between 0 and 1, is affected by the hydrophobicity (water sorptivity) and porosity of the material forming lamina 12. Water sorption and porosity exceeding about 60% will usually form a soft dispenser; whereas water sorption and porosity below about 60% usually provide a rigid dispenser.

The spatial distribution pattern of drug release from the exterior of dispenser 10 may be controlled through selection of the materials forming laminas 12 and 14. A localized flux in the vicinity of passageway 15 will occur if the water transport resistance, $R_3$, of lamina 12 is small relative to the water transport resistance, $R_1$, of lamina 14. Vice versa when $R_3$ is equal to or greater than $R_1$, drug release will be uniform over the entire exterior surface of lamina 12.

The materials used to make dispenser 10 are known materials and are exemplified by the following. Materials suitable for forming lamina 14 include the known homopolymers and copolymers that are employed as osmosis and reverse osmosis materials such as cellulose esters having a degree of substitution, D.S., on the anhydroglucose unit from greater than 0 up to 3 inclusive. The term "degree of substitution" means the average number of hydroxyl groups on the anhydroxyl groups on the anhydroglucose unit of the polymer replaced by a substituting group. Such polymers include cellulose acetate having a D.S. up to 1 and an acetyl content up to 21%; cellulose diacetate having a D.S. of 1 to 2 and acetyl content of 21% to 35%; cellulose triacetate having a D.S. of 2 to 3 and an acetyl content of 35% to 44.8%; cellulose propionate having a D.S. of 1.8 and a propionyl content of 38.5%; cellulose acetate propionate having an acetyl content of 1.5% to 7% and a propionyl content of 39% to 42%; cellulose acetate propionate having an acetyl content of 2.5% to 3% and an average combined propionyl content of 39.2% to 45% and a hydroxyl content of 2.8% to 5.4%; cellulose acetate butyrate having a D.S. of 1.8, an acetyl content of 13% to 15%, and a butyryl content of 34% to 39%; cellulose acetate butyrate having an acetyl content of 2% to 29.5%, a butyryl content of 17% to 53%, and a hydroxyl content of 0.5% to 4.7%; cellulose triacylates having a D.S. of 2.9 to 3 such as cellulose trivalerate, cellulose trilaurate, cellulose triheptylate, cellulose tricaprylate, cellulose trioctanoate, and cellulose tripropionate; cellulose diesters having a lower degree of substitution and prepared by the hydrolysis of the corresponding triester to yield cellulose diacylates having a D.S. of 2.2 to 2.6 such as cellulose disuccinate, cellulose dipalmitate, cellulose dioctanoate, cellulose dicaprylate and cellulose dipentanoate; and esters prepared from acyl anhydrides or acyl acids in an esterification reaction to yield esters containing different acyl groups attached to the same cellulose polymer such as cellulose acetate valerate, cellulose acetate succinate, cellulose propionate succinate, cellulose acetate octanoate, cellulose valerate palmitate, cellulose acetate palmitate and cellulose acetate heptanoate. The semipermeable materials useful for forming lamina 14 will have a water permeability of $10^{-5}$ to $10^{-1}$ (cc mil/cm$^2$ h bar), expressed per bar of hydrostatic or osmotic pressure difference across lamina 14 at the temperature of use while possessing a high degree of impermeability to drug formulation 17. The polymer forming lamina 14 may contain additives such as stabilizers, flux regulators, and plasticizers.

Microporous materials for making lamina 12 should be essentially inert, and should maintain their physical and chemical integrity during the dispensing period. They have a sponge-like appearance and may be isotropic (homogeneous structure throughout a cross-sectional area) or anisotropic (non-homogenous structure throughout a cross-sectional area). The pores of the material may be continuous pores that open on both faces of the lamina, or be interconnected through tortuous paths of regular or irregular shape. Generally, materials having a porosity of from 5% and 95%, a pore size of 10 angstroms to 100 microns, and an osmotic reflection coefficient less than 1, preferably greater than 0 to 0.5, may be used for making lamina 12. Microporous materials are commercially available and are made by etched nuclear tracking, by cooling a solution of flowable polymer below its freezing point whereby solvent evaporates from the solution in the form of crystals dispersed in the polymer and then curing the polymer followed by removing the solvent crystals, by cold or hot stretching at low or high temperatures until pores are formed, by leaching from a polymer a soluble component by an appropriate solvent, by ion exchange reaction, or by polyelectrolyte processes. Exemplary microporous materials for making lamina 12 are microporous polycarbonates comprised of linear polyesters of carbonic acid in which carbonate groups recur in the polymer chain, microporous materials prepared by the phosgenation of a dihydroxyl aromatic such as bisphenol, microporous poly(vinylchloride), microporous polyamides, such as polyhexamethylene adipamide, microporous modacrylic copolymers including those formed from vinylchloride and acrylonitrile and styrene and acrylonitrile, porous polysulfones characterized by diphenylene sulfone groups in a linear chain, halogenated poly(ethylene), polychloroethers, acetal polymers, polyesters prepared by esterification of a dicarboxylic acid or anhydride with an alkylene polyol, poly(alkylenesulfides), phenolic polyesters, microporous poly(saccharides), and microporous poly(saccharides) having substituted and unsubstituted anhydroglucose units.

Hydrogel materials useful for making lamina 12 include hydrogels that maintain their physical and chemical integrity in the environment of use and during the time agent is dispensed. The hydrogels useful for forming outer lamina 12 broadly include lightly cross-linked hydrophilic polymers. These polymers can have cross-links formed by covalent or ionic bonds. The hydrogels permit the passage of water and solutions, and they swell in the presence of water to a high degree without dissolution, usually exhibiting a 5 to 50 fold volume increase. The swelling of the hydrogels imparts a softness and gentle cushion response to touch in dispenser 10. These properties enhance the usefulness of hydrogels for forming lamina 12 of dispenser 10 as they increase the receptibility of dispenser 10 for use in cavities of the human body. The hydrogels should be non-toxic and non-irritating to the human body. Exemplary hydrogels include poly(hydroxyalkyl methacrylates), poly(acrylamide), poly(methacrylamide), poly(N - vinyl - 2 - pyrrolidone), anionic and cationic hydrogels, polyelectrolyte complexes, poly(vinylalcohol) having a low acetate residual and cross-linked with glyoxal, formaldehyde or glutaraldehyde, methylcellulose cross-linked with a dialdehyde, a cross-linked mixture of agar and sodium carboxymethylcellulose, a water-insoluble, water-swellable copolymer produced by forming a dispersion of finely divided copolymers of maleic anhydride with styrene, ethylene, propylene, butylene or isobutylene cross-linked with from about 0.001 to about 0.5 moles of a polyunsaturated cross-linking agent per mole of maleic anhydride in the copolymer as disclosed in US—A—3,989,586, water-swellable polymers of N-vinyl lactams as disclosed in US—A—3,992,562, semi-solid, cross-linked poly(vinyl pyrrolidone), cross-linked gelatin, cross-linked poly(acrylamide), diester cross-linked polyglucan hydrogels described in US—A—4,036,788, and ionogenic hydrophilic gels as described in *J. Biomedical Mater. Res.,* Volume 7, pages 123 to 136, 1973.

Lamina 13 may be made from various water soluble organic and inorganic compounds. Representative inorganic compounds that can be used for forming lamina 13 include magnesium chloride, sodium chloride, lithium chloride, potassium chloride, sodium carbonate, potassium sulfite, magnesium sulfate, calcium bicarbonate, sodium bicarbonate, potassium bicarbonate, sodium sulfite, potassium sulfite, lithium sulfite, magnesium sulfite, potassium acid phosphate, and sodium acid phosphate, and like. Typical organic compounds include carbohydrates such as glucose, sucrose, fructose, raffinose and lactose, and other organic water soluble compounds such as mannitol, inositol, urea, magnesium succinate, and tartaric acid.

The expression "active agent", as used herein, broadly includes any compound, composition of matter or mixture thereof, that can be delivered from the system to produce a beneficial and useful result. Exemplary active agents are pesticides, herbicides, germicides, biocides, algicides, rodenticides, fungicides, insecticides, anti-oxidants, plant growth promoters, plant growth inhibitors, preservatives, disinfectants, sterilization agents, catalysts, chemical reactants, fermentation agents, foods, food supplements, nutrients, cosmetics, drugs, vitamins, sex sterilants, fertility promoters, air purifiers, and micro-organism attenuators. As used herein the term "drug" means any physiologically or pharmacologically active substance that produces a local or systemic effect.

Dispenser 10 may be manufactured using standard pharmaceutical manufacturing techniques. For example, drug 17 and other ingredients that may be housed in compartment 16 and a solvent are mixed into a solid, semisolid, or gel formed by ballmilling, calendaring, stirring or rodmilling. The mixture is then pressed into a body of preselected shape. Semipermeable lamina 14 then is applied to the body by molding, spraying or dipping. Alternatively, lamina 14 can be cast into a film, shaped as desired and partly sealed to define compartment 16. The compartment is then filled with drug 17 and sealed. Dispenser 10 also can be manufactured empty of drug and then filled through passageway 15. Another, technique for forming lamina 14 is air suspension. That technique consists of suspending and tumbling the compressed body of drug in a current of air and entrained semipermeable material.

Lamina 13 may be applied by dipping, molding or air suspension techniques. Lamina 12 may be applied by the techniques used to apply lamina 14.

Examples

The following examples further illustrate the invention. The examples are not intended to limit the invention in any manner.

Example 1

A dispenser for administering potassium chloride orally was made as follows. 750 mg potassium chloride tablets (3.12 $cm^2$ in area) were compressed using a Manesty machine. 318 g of cellulose acetate having an acetyl content of 32% were homogeneously blended with 36 g of polyethylene glycol 400, and the blend was added to 7060 g of solvent. The solvent consisted of acetone:water, in the ratio of 88.5:11.5, wt:wt. The solvent and the ingredients were blended in a high shear blender for 25 to 30 minutes to yield an approximate 5% cellulose acetate solution. The blending was carried out at room temperature and atmospheric pressure. The solution and the potassium chloride tablets were placed in an Accela coater and the tablets were thus coated with the cellulose acetate. The coated tablets were dried in an air oven at 50°C for one week. The cellulose acetate coating was 125 microns thick. A 160 micron wide aperture was laser drilled through the cellulose acetate coating. 100 g of potassium chloride, 18 g of poly(vinyl pyrrolidone), and 1200 ml of water were blended and coated onto the cellulose acetate coated tablets by air suspension to a thickness of 60 microns (after drying). 65 g of cellulose acetate having an acetyl content of 32% was added to 41 g of sorbitol, 11.7 g of polyethylene glycol 400, and a solvent consisting of 1900 ml of acetone and 375 ml of water. All the ingredients were thoroughly blended in a high shear blender for 25 minutes and the blend was applied over the potassium chloride/poly(vinylpyrrolidone) coating by air suspension to a thickness of 120 microns.

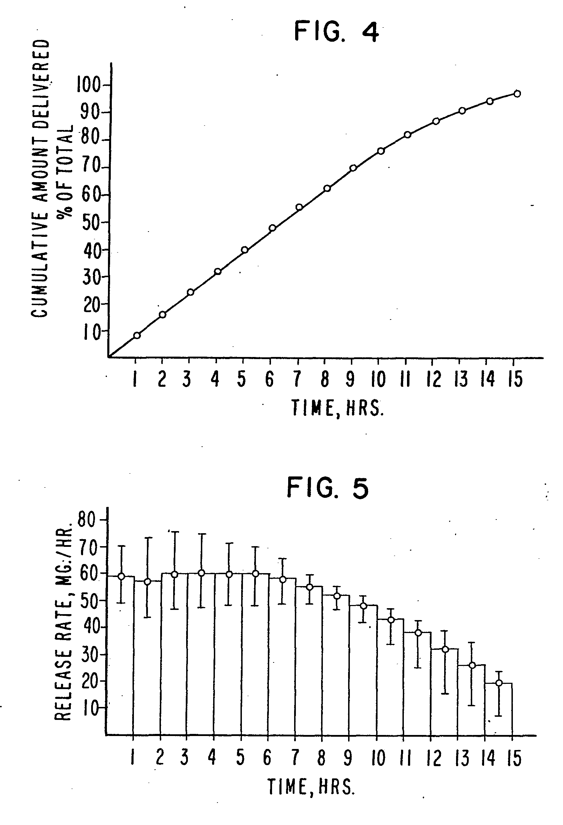

The resulting dispensers released potassium chloride at an average of 60 mg/h. Figure 4 shows the cumulative amount of potassium chloride delivered over a total time period by one of these dispensers. Figure 5 shows the average release rate in mg/h for eight of these dispensers.

Example 2

The procedure of Example 1 was repeated with the following differences: the tablets consisted of 187 mg of potassium chloride and 1 mg of magnesium stearate; the tablet area was 1.12 $cm^2$: the semipermeable lamina was formed from cellulose acetate having an acetyl content of 32%, with 401 g mixed in acetone-water solvent consisting of 8560 ml of acetone and 875 ml of water; the semipermeable lamina was 85 microns thick and the diameter of the aperture was 125 microns; the intermediate lamina was made from a composition consisting essentially of 132 g of potassium chloride and 33 g of poly(vinylpyrrolidone) in 2000 g water; the microporous lamina was formed from a composition consisting of 103 g of cellulose acetate having an acetyl content of 32% and 85 g sorbitol in a solvent consisting of 3050 ml of acetone and 600 ml of $H_2O$; and the microporous lamina was 100 microns thick.

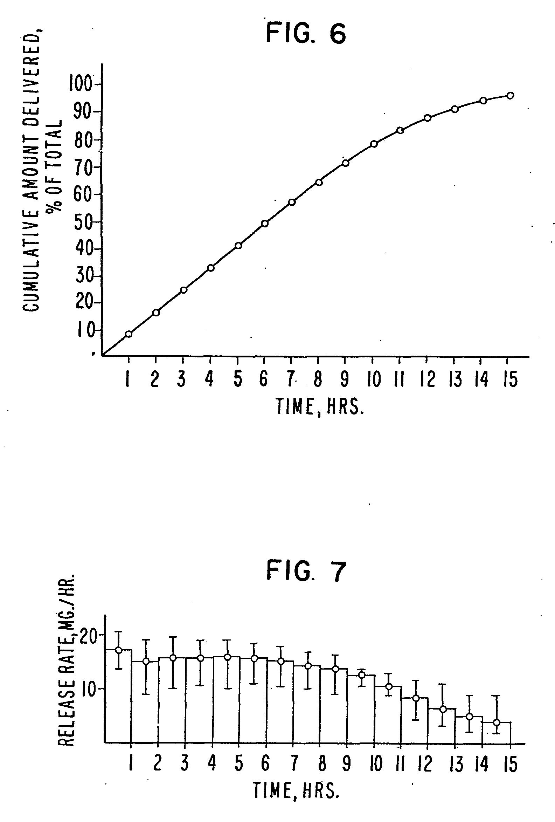

The dispensers prepared according to this example released potassium chloride at an average of 15 mg/h. Figure 6 shows the cumulative amount of potassium chloride delivered by one of over a 15 h period. Figure 7 shows the average rate of release for 10 dispensers.

Example 3

Dispensers designed as a suppository for rectal administration may be manufactured as follows: first, a conical shaped, stainless steel mold provided with a base portion, a tapered longitudinally extended, body portion, and a rounded lead portion is filled through an opening in the base with a composition comprising 750 mg of 8 - [(dimethylamino)methyl] - 7 - methoxy - 3 - methyl - 2 - phenyl - 4H - benzopyran - 4 - one hydrochloride and 2 mg of magnesium stearate and the composition is removed from the mold and coated with a semipermeable lamina as in Example 1 by air suspension. An aperture is laser drilled through the semipermeable lamina at the lead portion. Then, a layer of sucrose is coated onto the exterior surface of the semipermeable lamina and after drying, a microporous lamina is air suspension coated over the sucrose layer as in Example 1.

Example 4

Tablets of theophylline sodium glycinate are prepared as follows: Two kg of the drug are mixed with 100 g of poly(vinyl pyrrolidone) and the mix is converted to wet granules by mixing it with ethanol:water, (95.5% by volume, 450 ml). The wet granules are passed through a No. 40 sieve and dried in an oven at 50°C for 8 hrs. After drying, the granules are passed through a No. 30 sieve. Then 20 g of magnesium stearate are passed through a No. 40 sieve and mixed with the granules. The tablets are formed by compressing this mixture with a Manesty machine. Each tablet has an area of 3 cm$^2$.

A semipermeable lamina is formed by blending 11.05 g of hydroxypropyl cellulose, 62.05 g of cellulose acetate having an acetyl content of 32% and 8.50 g of polyethylene glycol 400, admixed with a solvent consisting of 1700 ml of methylene chloride and 700 ml of methanol, for 45 minutes at high shear. The tablets, each consisting of 600 mg of theophylline sodium glycinate, are coated with the hydroxypropyl cellulose-cellulose acetate-polyethylene glycol mix by air suspension in a Wurster coater. The coated tablets are dried for 24 hours at 50°C, and a 250 micron passageway is laser drilled through the coating.

An intermediate layer is then coated on the tablets from a mixture of 170 g of sorbitol, 67 g of poly(vinyl pyrrolidone), and 300 ml of water.

A microporous lamina is next coated on the tablets by dissolving 55 g of cellulose acetate having an acetyl content of 38.3% in 870 ml of methylene chloride and 500 ml of methanol (b) dissolving 45 g of sorbitol in 414 ml of methanol and 57 ml of water and then mixing (a) and (b) together at high shear; and (c) applying the mix of (a) and (b) by air suspension. The resulting dispensers release theophylline at a rate of 15 mg/h.

**Claims**

1. An osmotically driven dispenser (10) for dispensing an osmotically effective active agent composition (17) into a water containing environment comprising a water insoluble two layer laminated wall of an inner semipermeable lamina (14) and an outer microporous or hydrogel lamina (12), the wall defining an inner compartment (16), an osmotically effective active agent composition (17) contained within the compartment (16), and an outlet passageway (15) through the wall from which the active agent composition (17) is dispensed characterized in that the wall includes a third layer (13) of a water soluble composition located between the inner semipermeable lamina (14) and the outer microporous or hydrogel lamina (12) and the passageway (15) extends through the inner semipermeable lamina (14) from the compartment (16) to the third layer (13).

2. The osmotically driven dispenser (10) of claim 1 further characterized in that the third layer (13) is made of a carbohydrate or a salt.

**Revendications**

1. Dispositif (10) agissant par osmose pour l'apport d'une composition d'agent actif (17) "osmotiquement efficace" à un milieu environnant contenant de l'eau, qui comprend une paroi stratifiée à deux couches insolubles dans l'eau, à savoir une couche interne semi-perméable (14) et une couche externe microporeuse ou d'hydrogel (12), la paroi délimitant un compartiment interne (16) qui contient une composition d'agent actif osmotiquement efficace (17), et un passage (15) vers l'extérieur pratiqué à travers la paroi, par lequel la composition d'agent actif (17) est libérée, dispositif caractérisé en ce que la paroi comporte une troisième couche (13) d'une composition hydrosoluble placée entre la couche interne semi-perméable (14) et la couche externe microporeuse ou d'hydrogel (12), et le passage (15) s'étend à travers la couche interne semi-perméable (14), du compartiment (16) à la troisième couche (13).

2. Dispositif selon la revendication 1, caractérisé en ce que la troisième couche (13) est une couche d'un glucide ou d'un sel minéral.

**Patentansprüche**

1. Durch Osmose wirksame Abgabevorrichtung (10) zur Abgabe eines osmotisch wirksamen aktiven Mittels (17) an eine wasserhaltige Umgebung, umfassend eine wasserunlösliche aus zwei laminierten Schichten bestehende Wand, aus einer inneren semipermeablen Schicht (14) und einer äußeren mikroporösen oder Hydrogelschicht (12), wobei die Wand eine innere Kammer (16) umschließt, ein osmotisch wirksames aktives Mittel (17), das in der Kammer (16) enthalten ist, und eine Ausgangsöffnung (15) durch die Wand, aus der das wirksame Mittel (17) abgegeben wird, dadurch gekennzeichnet, daß die Wand eine dritte Schicht (13) aus einer wasserlöslichen Masse umfaßt, die sich zwischen der inneren semipermeablen Schicht (14) und der äußeren mikroporösen oder Hydrogelschicht (12) befindet, und der Durchgang (15) sich durch die innere semipermeable Schicht (14) von der Kammer (16) nach der dritten Schicht (13) hin erstreckt.

2. Durch Osmose wirksame Abgabevorrichtung (10) nach Anspruch 1, ferner dadurch gekennzeichnet, daß die dritte Schicht (13) aus einem Kohlenhydrat oder einem Salz besteht.

FIG. 1

FIG. 2

FIG. 3

# FIG. 4

Graph: CUMULATIVE AMOUNT DELIVERED % OF TOTAL vs TIME, HRS.

# FIG. 5

Graph: RELEASE RATE, MG./HR. vs TIME, HRS.

## FIG. 6

## FIG. 7